(19) Europäisches Patentamt
European Patent Office
Office européen des brevets

(11) Publication number: **0 027 835**
**A1**

(12) # EUROPEAN PATENT APPLICATION

(21) Application number: **79302262.5**

(22) Date of filing: **18.10.79**

(51) Int. Cl.³: **C 07 D 311/58**, C 07 D 311/68, C 07 D 491/052 // (C07D491/052, 203/00, 311/00)

(43) Date of publication of application: **06.05.81**
**Bulletin 81/18**

(84) Designated Contracting States: **BE CH DE FR GB IT NL SE**

(71) Applicant: **WARNER-LAMBERT COMPANY, 201 Tabor Road, Morris Plains, New Jersey 07950 (US)**

(72) Inventor: **Parcell, Robert Ford, 1538 Mockingbird Lane, Lakeland Florida 33801 (US)**
Inventor: **Nordin, Ivan Conrad, 5770 Warren Road, Ann Arbor Michigan 48105 (US)**

(74) Representative: **Jones, Michael Raymond et al, Haseltine Lake & Co. 28 Southampton Buildings Chancery Lane, London WC2A 1AT (GB)**

(54) **Process for producing cis-(+,-)-3,4-dihydro-N,N,2-trimethyl-2H-1-benzopyran-3-amine, intermediates produced thereby and their preparation.**

(57) A process for the preparation of cis-(±)-3,4-dihydro-N,N,2-trimethyl-2H-1-benzopyran-3-amine (I), a known anti-depressant, is disclosed. The process of the invention is a multi-step process starting with 2-methyl-2H-1-benzopyran (II), which involves the production of the novel intermediates (2 alpha, 3 beta, 4 alpha)-(±)-3,4-dihydro-2-methyl-4-(methylamino)-2H-1-benzopyran-3-ol (IV) and its hydrogen sulphate ester (V), as well as (1a alpha, 2 beta, 7b alpha)-(±)-1,1a,2,7b-tetrahydro-1,2-dimethyl-[1]benzopyrano-[3,4-b]azirine (VI) and cis-(±)-3,4-dihydro-N,2-dimethyl-2H-1-benzopyran-3-amine (VII). In the final step of the process, the novel compound VII is methylated to form the desired compound I. The process is less expensive, less hazardous and less difficult than known ways of producing compound I.

Compounds IV and V have the formula:

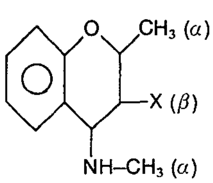

where X is $-OH$ and $-OSO_3H$ respectively.

Compound VI has the formula:

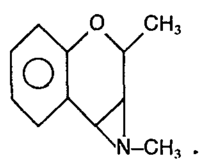

Compound VII has the formula:

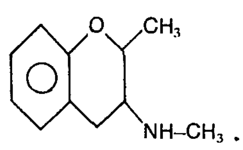

EP 0 027 835 A1

0027835

TITLE MODIFIED
see front page



# PROCESS FOR PRODUCING CIS-(±)-3,4-DIHYDRO-N,N,2-TRIMETHYL-2H-1-BENZOPYRAN-3-AMINE AND INTERMEDIATES PRODUCED THEREBY

This invention relates to a process for the production of cis-(±)-3,4-dihydro-N,N,2-trimethyl-2H-1-benzopyran-3-amine of the following formula (I), and to certain intermediates used in, and produced in, that production:

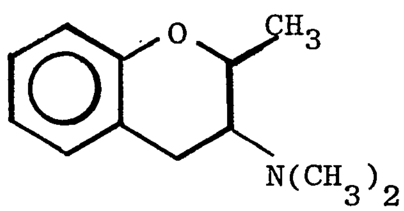

(cis)

I

As disclosed by Lockhart, I.M., in United States Patent No. 3,607,886, N,N,2-trimethyl-3-chromanamine, alpha-isomer (which is another name for the cis-(±)-3,4-dihydro-N,N,2-trimethyl-2H-1-benzopyran-3-amine designated compound I in the present invention) exhibits anti-depressant activity. According to the United States Patent No. 3,607,886, N,N,2-trimethyl-3-chromanamine, alpha-isomer and its acid-addition salts may be produced by reacting 2-methyl-3-nitro-2H-1-benzopyran with lithium aluminium hydride and hydrolyzing the resulting product to obtain a mixture of 2-methyl-3-chromanamine,alpha-isomer and beta-isomer, from which the alpha-isomer is subsequently separated by

a precipitation and recrystallization process. The alpha-isomer is then methylated to obtain the desired N,N,2-trimethyl-3-chromanamine.

In the United States Patent No. 3,629,289, a related Lockhart patent, the alpha-isomer of the 2-methyl-3-chromanamine intermediate and the process for its production are more fully described.

Bachman, et al., in J. Am. Chem. Soc. 70: 599-601 (1948) disclose the preparation of 2-methyl-3-aminochroman by hydrogenation of 2-methyl-3-nitro-1,2-benzopyran using a Raney nickel catalyst, the term "aminochroman" being interchangeable with the term "chromanamine".

In the aforementioned United States Patents Nos. 3,607,886 and 3,629,289, the distinction between the alpha-and beta-isomeric forms of the compounds prepared is discussed, particularly with respect to the physiochemical and pharmacological properties.

Lovgren, K., et al., in Acta. Pharm. Suecica 14: 21-29 (1977) disclose the preparation of certain cis and trans 3-hydroxy-4-isopropylaminochromans starting with 6-methoxy-2H-chromen. Lovgren, et al., recognize that two completely different syntheses are required to obtain the cis and trans isomeric configurations. Intermediates formed during the synthesis of the trans isomers include trans-3,4-dibromo-6-methoxychroman and trans-3-bromo-4-hydroxy-6-methoxychroman. However, as none of the Lovgren compounds have a 2-methyl substituent, this reference is concerned only with isomerism at the 3,4-position of the chroman ring. In the present invention, the 2-methyl substituent presents additional isomeric possibilities which are not considered in the Lovgren, et al., reference.

The preparation, isomeric configuration and pharmacological properties of related aminochroman derivatives are discussed in the following references: Huckle, et al., J. Med. Chem. 12: 277-279 (1969);

Lockhart, et al., J. Med. Chem. 15: 863-865 (1972); Sarda, et al., C.R.Acad. Sc. Paris 279: 281-4 (1974); Sarda, et al., Eur. J. Med. Chem. 11: 251-257 (1976); and Sarda, et al., Eur. J. Med. Chem. 11: 257-262 (1976).

In Japanese Application No. 52-083-829 (Takeda Chemical Industries) ring opening of an azirine derivative is achieved by hydrolysis: thus 5,6-dihydroxy-1,1-alpha, 2,3,4,8b-hexahydrobenzo[3,4]cyclohepta[1,2-b]azirine is hydrolyzed to form 1,2,5-trihydroxy-6-isopropylamino-6,7,8,9-tetrahydro-5H-benzocycloheptene hydrobromide, a useful remedy for asthma. The azirine derivative of the sixth reaction step of the main overall process of the present invention involves a different ring system,and the ring opening is, instead, achieved by means of catalytic hydrogenation.

According to one aspect of the present invention, there is provided a process for producing cis-(±)-3,4-dihydro-N,N,2-trimethyl-2H-1-benzopyran-3-amine having the formula I:

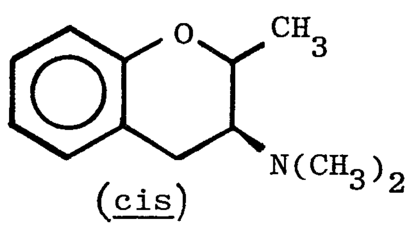

(cis)

I

or a non-toxic pharmaceutically acceptable salt thereof, which process comprises the following steps:

1. reacting a solution of 2-methyl-2H-1-benzopyran having the formula II:

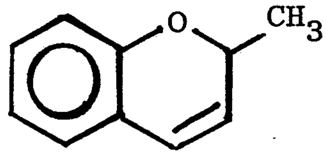

II

in water and/or in a non-reactive, water -miscible solvent with bromine to obtain, in situ, 3,4-dibromo-3,4-dihydro-2-methyl-2H-1-benzopyran having the formul IIa:

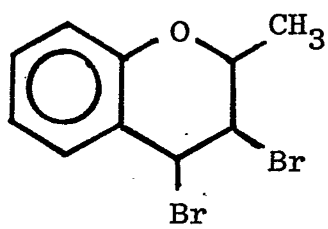

IIa

2. treating the reaction mixture produced in Step 1 with water and neutralizing with an alkali to obtain 3,4-dihydro-3-bromo-2-methyl-2H-1-benzopyran-4-ol having the formula III:

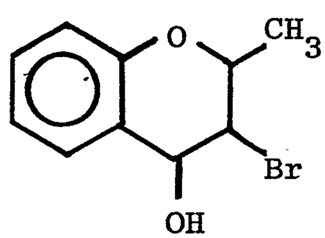

III

3. reacting compound III in water or a water-miscible solvent, with an aqueous solution of methylamine to form, in situ, 3,4-epoxy-2-methylbenzopyran having the formula IIIa:

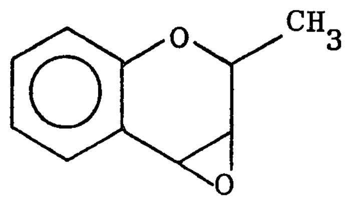

IIIa

and continuing the reaction to form (2 alpha, 3 beta, 4 alpha)-(±)-3,4-dihydro-2-methyl-4-(methylamine)-2H-1-benzopyran-3-ol having the formula IV:

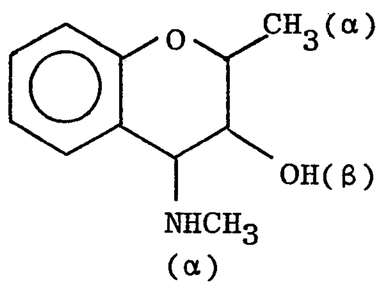

IV

4. reacting sulphuric acid in an organic solvent with compound IV at a temperature above 110°C to obtain (2 alpha, 3 beta, 4 alpha)-(±)-3,4-dihydro-2-methyl-4-(methylamino)-2H-1-benzopyran-3-ol, hydrogen sulphate ester having the formula V:

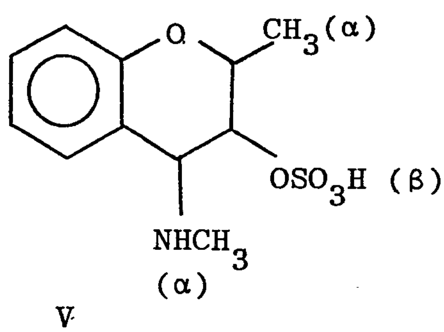

V

5. heating compound V and an alkaline metal hydroxide to obtain (1a alpha, 2 beta, 7b-alpha)-(±)-1,1a,2,7b-tetrahydro-1,2-

dimethyl[1]benzopyrano[3,4-b]azirine having the formula VI:

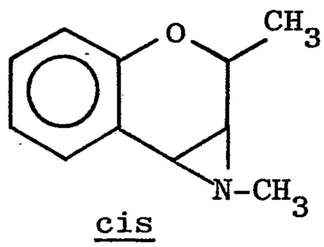

cis

VI

6. subjecting a solution of compound VI to catalytic hydrogenation to effect ring opening and formation of cis-(±)-3,4-dihydro-N,2-dimethyl-2H-1-benzopyran-3-amine having the formula VII:

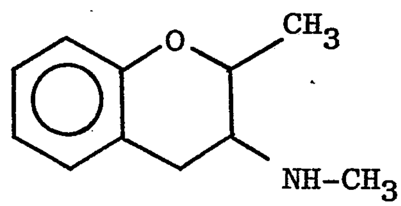

cis

VII

and,

7. methylating a solution of compound VII to obtain compound I in the form of the free base or a non-toxic pharmaceutically acceptable acid-addition salt thereof.

In step 1 of the process the 2-methyl-2H-1-benzopyran (II) is in water and/or in a non-reactive, water-miscible solvent, and is preferably in both water and a non-reactive, water-miscible solvent. Also, in step 1 of the process, the preferred solvent (when present) is tetrahydrofuran, and preferably the temperature of the reaction is maintained at approximately $7^{o}C-10^{o}C$ for about 45 minutes.

In step 2 of the process of the present invention, the

reaction mixture containing compound IIa is treated with water and preferably heated at reflux temperature for from 5 minutes to 3 hours, followed by neutralization with an alkali, for instance aqueous sodium hydroxide solution, to obtain compound III.

In step 3 of the process of the present invention compound III dissolved in water or a different water-miscible solvent is reacted with an aqueous solution of methylamine, preferably while maintaining the temperature below 25°C to form, in situ, compound IIIa. Examples of suitable water-miscible solvents for this step are lower alkanols, for example methanol and ethanol; water-miscible ethers, for example diglyme and dioxane; and mixtures thereof. The preferred solvent is methanol. Generally, at least 2 molar equivalents (moles) of methylamine per mole of compound III are required, with a moderate to large excess of methylamine being preferred.

As the second part of step 3 of the process according to the present invention, the reaction mixture containing compound IIIa is preferably refluxed for from one hour to 48 hours; more preferably this phase of the reaction is conducted at from 20°C to 30°C for from 16 to 30 hours. The product of the reaction, compound IV, may be isolated by conventional means, for example by evaporation, followed by crystallization.

In step 4 of the process according to the present invention, sulphuric acid dissolved in an organic solvent, preferably a hydrocarbon solvent or a halogenated hydrocarbon solvent, reacts with compound IV to produce compound V. Examples of suitable organic solvents for use in step 4 are hydrocarbons, for example toluene and xylene; chlorinated hydrocarbons, for example tetrachloroethane and chlorobenzene; and mixtures thereof. The preferred solvent is xylene. The reaction mixture is conveniently heated at reflux under a water separator until water is no longer collected. Reflux

temperatures above 110°C are desirable. At 135°C to 145°C, the reaction usually goes to completion in about one to three hours. A slight excess of either reactant may be used, but approximately equimolar amounts of reactants are preferred. Compound V may be isolated by conventional means, for example crystallization.

In step 5 of the process according to the present invention, compound V is heated with an alkaline metal hydroxide, that is either an alkali metal hydroxide (i.e. a hydroxide of a Group IA metal) or an alkaline earth metal hydroxide (i.e. a hydroxide of a Group IIA metal). Preferably there is used a strong base i.e. an alkali metal hydroxide, more preferably sodium hydroxide. At least two molar equivalents of alkaline metal hydroxide are usually required per mole of sulphate ester V, with an excess being preferred. While additional organic solvent is unnecessary in this step of the process, a two phase system of an aqueous alkaline solution and a water-immiscible, non-reactive organic solvent is desirable; toluene is the preferred organic solvent. The temperature of the reaction is not critical but, when toluene is used as the organic solvent, a temperature of from 95°C to 100°C and a reaction duration of 2 to 4 hours are preferred. Compound VI may be isolated by conventional means, for example evaporation of the organic phase.

In step 6 of the process according to the present invention, a solution of compound VI is subjected to catalytic hydrogenation to effect ring opening and formation of compound VII: examples of solvents suitable for producing the solution of compound VI are hydrocarbon solvents, for example benzene, toluene and xylene; lower alkanols, for example methanol and ethanol; ethers, for example tetrahydrofuran, dioxane and diglyme; and mixtures thereof. For the hydrogenation, there may, for instance, be used noble metal catalysts such as platinum or palladium, and oxides thereof, optionally supported on a carbonaceous carrier such as charcoal.

The preferred hydrogenation catalyst is palladium on a charcoal carrier. Hydrogen pressure during the hydrogenation is not critical and pressures of from 15 to 150 pounds per square inch (103.4 to 1034 kPa), are suitable, The hydrogenation is normally carried out at ambient temperature until no further hydrogen is taken up. Generally, one mole of hydrogen is required for complete conversion of one mole of the azirine compound VI to compound VII. Compound VII may be isolated by standard procedures, for example by evaporation of solvent. Compound VII may also be isolated as a salt by reaction of the base with a suitable acid.

In step 7 of the process according to the present invention, a solution of compound VII is methylated to obtain the desired compound I. Suitable methylating agents include, for instance, methyl halides, particularly methyl iodide; dimethyl sulphate; methyl sulphonates, such as methyl methanesulphonate and methyl p-toluenesulphate; mixtures of formaldehyde and formic acid; formaldehyde and hydrogen in the presence of a noble metal catalyst; and trimethyloxonium salts, such as trimethyloxonium tetrafluoroborate, trimethyloxonium tetrachloroferrate, trimethyloxonium hexafluoroantimonate and trimethyloxonium tetrachloroaluminate. When using the methyl halides, the methyl sulphates, dimethyl sulphate or trimethyloxonium salts as the methylating agent, the reaction is preferably carried out in the presence of a base, for example potassium carbonate, sodium carbonate or sodium bicarbonate. The preferred methylating agent is a mixture of formaldehyde and formic acid.

When using the preferred formaldehyde-formic acid methylation procedure, excess formic acid can serve as the solvent for the reaction and additional solvent is neither necessary nor desirable. With other methylating agents, a variety of solvents may be used to produce the solution of compound VII; these include hydrocarbons, for instance

benzene, toluene and xylene; ethers, for example diethyl ether, dioxane, tetrahydrofuran and diglyme; lower alkanones, for instance acetone and 2-butanone (with the exception that lower alkanone solvents are not used in hydrogenations with formaldehyde); tertiary amides, for example dimethylformamide and N-methyl-2-pyrrolidinone; and mixtures thereof.

The temperature and duration of the methylation reaction of step 7 are not critical. In general, but not necessarily, the reaction may be carried out at 10 to 150°C or at reflux of the solvent, for from 48 hours to 20 minutes. Longer reaction times are necessary when lower temperatures are utilized. Using the preferred formaldehyde-formic acid methylation procedure, a temperature of from 40°C to 100°C for from 6 hours to 30 minutes is usually suitable. Generally, one mole of methylating agent is required per mole of primary amine (VII). When using the preferred formaldehyde-formic acid methylation procedure, a substantial excess of methylating agent is desirable. The final product, compound I, may be isolated as the free base or as a salt by standard procedures.

The starting material, 2-methyl-2H-1-benzopyran (compound II) used in the novel process of the present invention is a known compound and is described by Ursula Koch-Pomeranz, et al., in Helv. Chim. Acta. 56: 2981 at 2986 (1973) wherein this starting material is prepared starting from 1'-methylpropargyl phenyl ether.

The novel process of the present invention is an improvement over previously known techniques for producing compound I in that certain hazardous intermediates required in the prior art techniques are avoided in the present invention. In addition, there is a substantial reduction in costs of starting materials and in the processing technique required.

The final compound I, prepared according to the process of the present invention, is described in the

aforementioned United States Patent No. 3,607,886 as having anti-depressant activity as evidenced by positive results obtained in certain pharmacological assays: compound I has been found to cause a significant suppression of hyper-irritability in rats following doses of 12.5 to 100 mg/kg, administered intraperitoneally; and compound I was found to suppress the mouse-killing instinct in killer rats when administered at a dose of 15 mg/kg, administered intraperitoneally.

Thus, compound I is indicated in the management of certain mental states such as depression when administered parenterally or orally to mammals.

For administration, coupound 1 may be formulated according to methods well known to the pharmacists' art. Typically, saline solutions of compound I are used for injectable purposes. For oral administration, cmpound I may be administered in a saline solution or may be formulated with excipients such as lactose into tablets suitable for oral administration.

Other aspects of the present invention include:-

the process for producing compound VII from compound II using steps 1 to 6;

the process for producing compound I from compound IV using steps 4 to 7;

the process for producing compound VII from compound IV using steps 4 to 6;

the process for producing compound VII from compound VI using step 6;

the process for producing compound VI from compound II using steps 1 to 5;

the process for producing compound V from compound II using steps 1 to 4;

the process for producing compound IV from compound II using steps 1 to 3;

the process for producing compound I from compound VII using step 7; and

the novel compounds IV, V, VI and VII each of

which is useful as an intermediate in one or more of the aforementioned process aspects of the present invention.

In order further to illustrate the present invention, the following Examples are provided.

EXAMPLE 1

(2 alpha, 3 beta, 4 alpha)-(±)-3,4-Dihydro-2-methyl-4-(methylamino)-2H-1-benzopyran-3-ol.

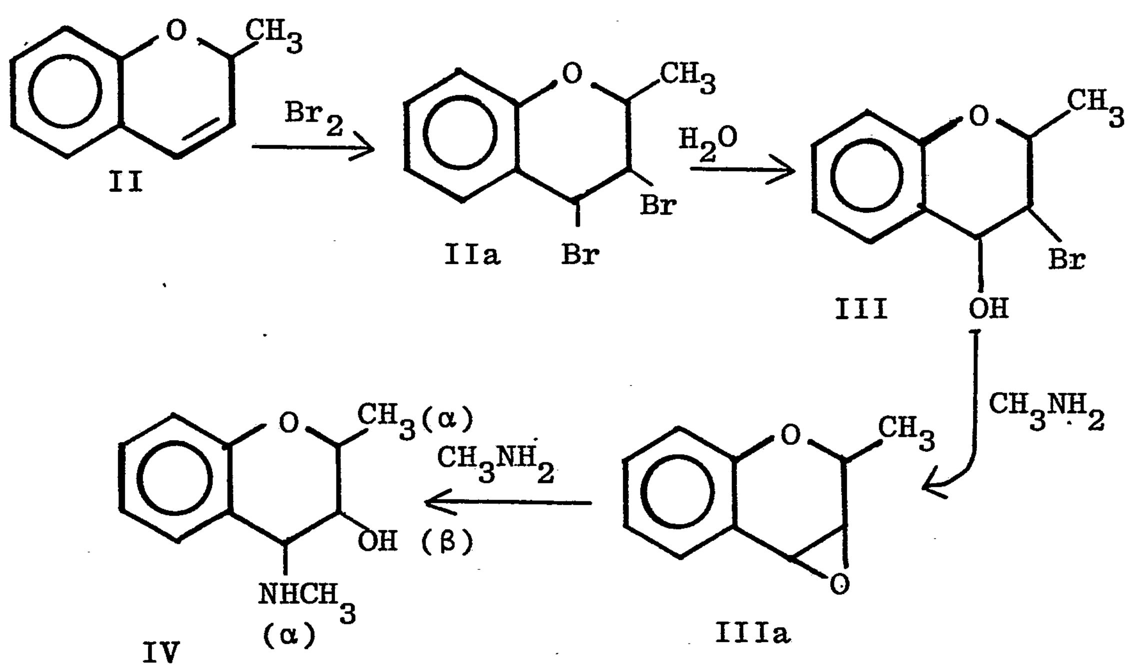


II IIa III IIIa IV

A stirred solution of 149.3 g of 2-methyl-2H-1-benzopyran (II) in 750 ml of tetrahydrofuran and 72 ml of water was cooled to 8°C and treated dropwise with 162 g of bromine over 45 minutes while maintaining the temperature in the range 7-10°C. There were then added 144 ml of water and the solution was stirred and heated at reflux for 1 hour. The stirred solution was cooled and treated with 64 g of 50% aqueous sodium hydroxide solution while maintaining the temperature in the range 10-15°C. The resulting aqueous phase was separated off and discarded. The organic phase was treated with 100 ml of water and the mixture was stirred and heated at reflux for 2.75 hours. The mixture was then evaporated at reduced pressure to remove

tetrahydrofuran, and the aqueous residue of 3,4-dihydro-3-bromo-2-methyl-2H-1-benzopyran-4-ol (melting point, $77^o$-$79^oC$) was dissolved in 600 ml of methanol. The methanol solution was stirred and treated slowly with 300 ml of 40% aqueous methylamine while maintaining the temperature below $25^oC$. The resulting solution was stirred slowly at $20^o$-$25^oC$ for 24 hours, heated at reflux for 1 hour and evaporated at reduced pressure to remove methanol. The aqueous residue was treated with 100 g of sodium hydroxide and the mixture was extracted with three 200 ml portions of toluene. The toluene extracts were combined and the solution was extracted with excess 6<u>N</u> hydrochloric acid. The aqueous acid extract was made alkaline with excess 50% aqueous sodium hydroxide solution, and extracted with three 200 ml portions of toluene. The toluene extracts were combined and the solution was treated with activated charcoal and anhydrous magnesium sulphate, and filtered. The filtrate was evaporated at reduced pressure and the residue of (2 alpha, 3 beta, 4 alpha)-(±)-3,4-dihydro-2-methyl-4-(methylamino)-2H-1-benzopyran-3-ol was crystallized from xylene; mp 110-$113^oC$.

EXAMPLE 2

(2 alpha, 3 beta, 4 alpha)-(±)-3,4-Dihydro-2-methyl-4-(methylamino)-2H-1-benzopyran-3-ol, hydrogen sulphate ester

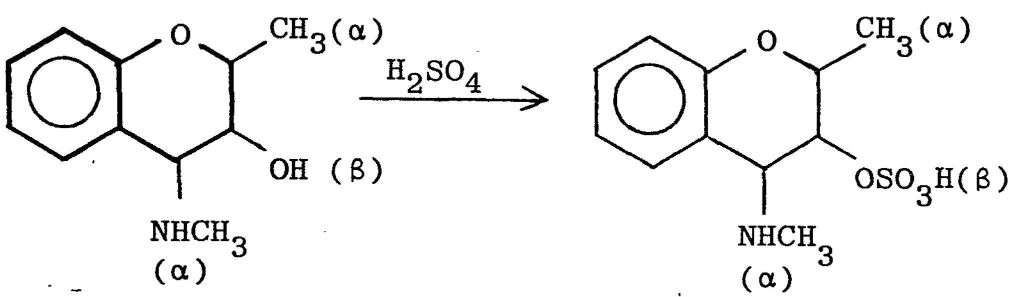


IV

V

A mixture of 54.5 g of 96% suphuric acid and 800 ml of xylene was stirred vigorously and treated portionwise with 103 g of finely divided (2 alpha, 3 beta, 4 alpha)-(±)-3,4-dihydro-2-methyl-4-(methylamino)-2H-1-benzopyran-3-ol (as produced in Example 1) over a period of 10 minutes. The mixture was stirred and heated at reflux under a water separator until no more water was collected (about 2.5 hours). The mixture was cooled to 10°C and the precipitate of (2 alpha, 3 beta, 4 alpha)-(±)-3,4-dihydro-2-methyl-4-(methylamino)-2H-1-benzopyran-3-ol, hydrogen sulphate ester, was collected by filtration, washed with xylene and then with pentane, and dried at reduced pressure; mp 246-247°C.

EXAMPLE 3

(1a alpha, 2 beta, 7b alpha)-(±)-1,1a,2,7b-Tetrahydro-1,2-dimethyl[1]benzopyrano[3,4-b]azirine.

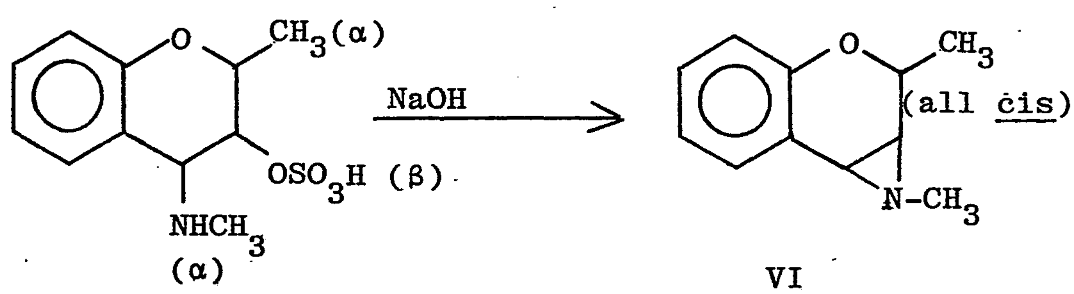


V

VI

A mixture of 144 g of (2 alpha, 3 beta, 4 alpha)-(±)-3,4-dihydro-2-methyl-4-(methylamino)-2H-1-benzopyran-3-ol, hydrogen sulphate ester (as produced in Example 2), and 1 l. of 2N sodium hydroxide was stirred as the solid dissolved, followed by precipitation of the sodium salt. A nitrogen atmosphere was then provided, 200 ml of toluene were added, and the mixture was heated at 97-98°C for 3 hours. The organic phase was separated off, and the aqueous phase was extracted twice with 200 ml portions of toluene. The combined toluene extracts were dried over anhydrous potassium carbonate, treated with activated charcoal and filtered. The toluene filtrate, containing (1a alpha, 2 beta, 7b alpha)-(±)-1,1a,2,7b-tetrahydro-1,2-dimethyl[1]-

benzopyrano[3,4-b]azirine, may be used as such without isolation of the product. If desired, however, the azirine may be isolated by evaporation of the toluene followed by purification by distillation; bp 86 -88°C/0.12 mm.

EXAMPLE 4

cis-(±)-3,4-Dihydro-N,2-dimethyl-2H-1-benzopyran-3-amine

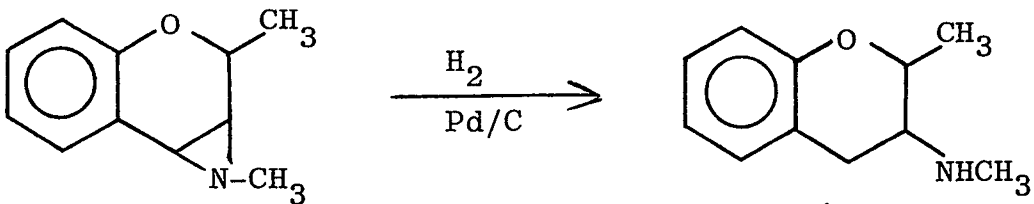


VI (all cis)

cis VII

The toluene solution of (1a alpha, 2 beta, 7b alpha) -(±)-1,1a,2,7b-tetrahydro-1,2-dimethyl[1]-benzopyrano [3,4-b]azirine, obtained in Example 3, was treated with 1 g of 20% palladium on charcoal and the mixture was shaken with hydrogen at 50 pounds per square inch (344.7kPa) until hydrogen uptake ceased (about 1 hour). The catalyst was removed by filtration and the filtrate was evaporated at reduced pressure. The residue is triturated with an equal volume of diethyl ether and the mixture was chilled and filtered to remove a small amount of insoluble material. The ethereal solution was evaporated at reduced pressure to give cis-(±)-3,4-dihydro-N,2-dimethyl-2H-1-benzopyran-3-amine, suitable for use without further purification.

The hydrochloride salt, prepared from the free base compound and dry hydrogen chloride in 2-propanol and precipitated with diethyl ether, melted at 238.5-240°C. The hydrobromide salt, prepared similarly, melted at 219-220°C.

EXAMPLE 5

cis-(±)-3,4-Dihydro-N,N,2-trimethyl-2H-1-benzopyran-3-amine, monohydrochloride.

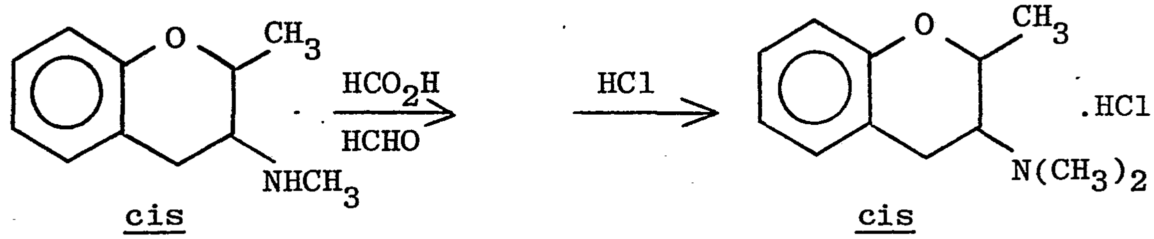


cis VII

cis I

A mixture of 64 ml of 37% aqueous formaldehyde and 135 ml of 97-100% formic acid was cooled to 10°C and treated slowly with stirring with 88.4 g of cis-(±)-3,4-dihydro-N,2-dimethyl-2H-1-benzopyran-3-amine (produced as in Example 4) while maintaining the temperature below 15°C. The mixture was stirred and heated at 45-50°C for 20 minutes, then heated to 90°C until carbon dioxide evolution ceased (about 15 minutes), and then cooled to 35°C and treated with 55 ml of concentrated hydrochloric acid and 100 ml of methanol. The resulting mixture was stirred for 30 minutes, and then evaporated at reduced pressure. The crystalline residue was dissolved in 1.2 l. of hot 2-propanol and the solution was treated with activated charcoal and filtered. The filtrate was concentrated to a volume of 800 ml and chilled. The resulting crystalline precipitate of cis-(±)-3,4-dihydro-N,N,2-trimethyl-2H-1-benzopyran-3-amine monohydrochloride was collected by filtration, washed with cold 2-propanol and dried; mp 215-216°C, after recrystallization from ethanol using a charcoal treatment.

The term "lower" as used in this specification in relation to the compounds alkanols and alkanones means alkanols and alkanones containing a maximum of 4 carbon atoms.

CLAIMS

1. A process for producing cis-(+)-3,4-dihydro-N,N,2-trimethyl-2H-1-benzopyran-3-amine having the formula I:

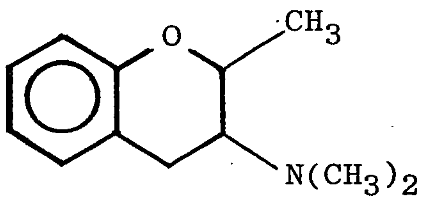

(cis)

I

or a non-toxic pharmaceutically acceptable salt thereof, which process comprises the following steps:

1. reacting a solution of 2-methyl-2H-1-benzopyran having the formula II:

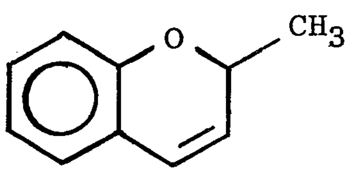

II

in water and/or in a non-reactive, water-miscible solvent, with bromine to obtain, in situ, 3,4-dibromo-3,4-dihydro-2-methyl-2H-1-benzopyran having the formula IIa:

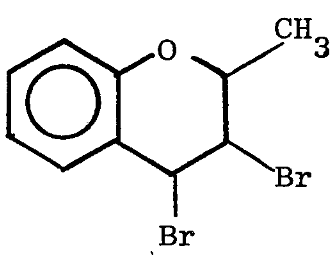

IIa

2. treating the reaction mixture produced in step 1 with water and neutralizing with an alkali to obtain 3,4-dihydro-3-bromo-2-methyl-2H-1-benzopyran-4-ol having the formula III:

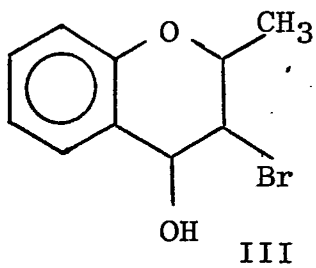

III

3. reacting compound III in water or a water-miscible solvent, with an aqueous solution of methylamine to form, in situ, 3,4-epoxy-2-methylbenzopyran having the formula IIIa:

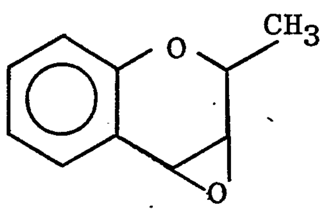

IIIa

and continuing the reaction to form (2 alpha, 3 beta, 4 alpha)-($\pm$)-3,4-dihydro-2-methyl-4-(methylamine)-2H-1-benzopyran-3-ol having the formula IV:

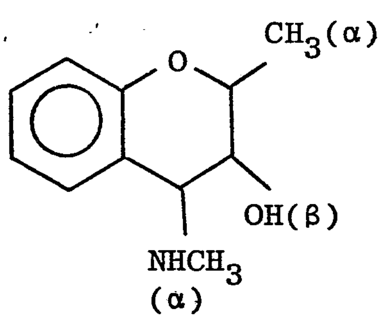

IV

4. reacting sulphuric acid in an organic solvent with compound IV at a temperature above 110°C to obtain (2 alpha, 3 beta,-4alpha)-(±)-3,4-dihydro-2-methyl-4-(methylamino)-2H-1-benzopyran-3-ol, hydrogen sulphate ester having the formula V:

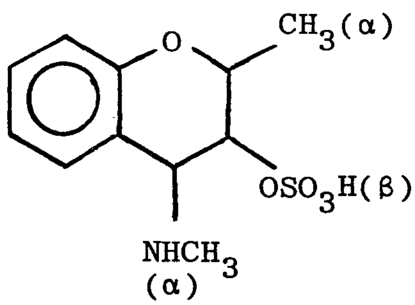

V

5. heating compound V and an alkaline metal hydroxide to obtain (1a alpha, 2 beta, 7b-alpha)-(±)-1,1a,2,7b-tetrahydro-1,2-dimethyl [1] benzopyrano [3,4-b]azirine having the formula VI:

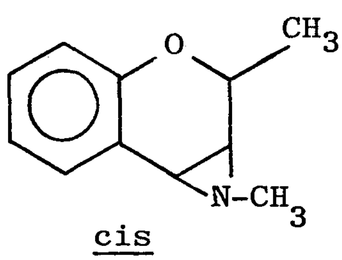

cis

VI

6. subjecting a solution of compound VI to catalytic hydrogenation to effect ring opening and formation of cis-(±)-3,4-dihydro-N,2-dimethyl-2H-1-benzopyran-3-amine having the formula VII:

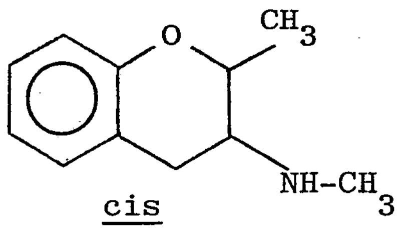

cis

VII

and,

7. methylating a solution of compound VII to obtain compound I in the form of the free base or a non-toxic pharmaceutically acceptable acid-addition salt thereof.

2. A process for producing cis-(+)-3,4-dihydro-N,2-dimethyl-2H-1-benzopyran-3-amine having the formula VII:

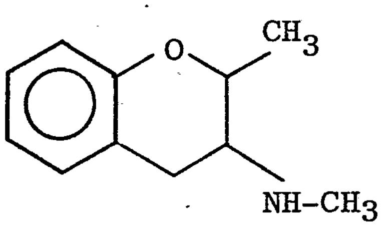

cis

VII

which process comprises the following steps:

1. reacting a solution of 2-methyl-2H-1-benzopyran having the formula II:

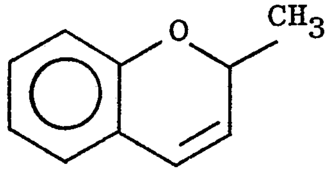

in water and/or in a non-reactive, water-miscible solvent, with bromine to obtain, in situ, 3,4-dibromo-3,4-dihydro-2-methyl-2H-1-benzopyran having the formula IIa:

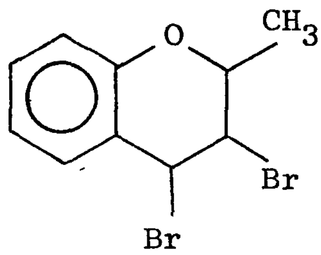

IIa

2. treating the reaction mixture produced in step 1 with water and neutralizing with an alkali to obtain 3,4-dihydro-3-bromo-2-methyl-2H-1-benzopyran-4-ol having the formula III:

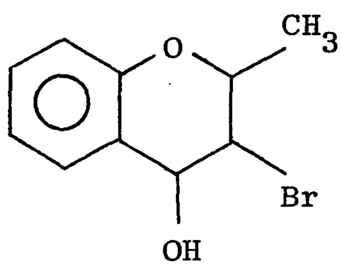

III

3. reacting compound III in water or a water--miscible solvent, with an aqueous solution of methylamine to form, in situ, 3,4-epoxy-2-methylbenzopyran having the formula IIIa:

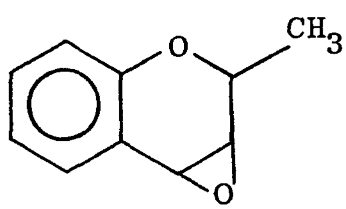

and continuing the reaction to form (2 alpha, 3 beta, 4 alpha)-(±)-3,4-dihydro-2-methyl-4-(methylamino)-2H-1-benzopyran-3-ol having the formula IV:

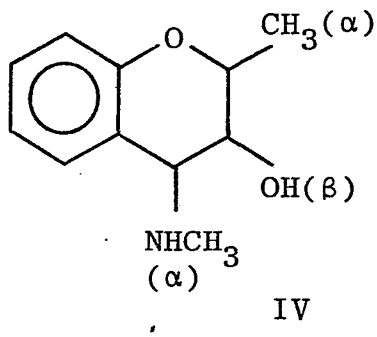

IV

4. reacting sulphuric acid in an organic solvent with compound IV at a temperature above 110$^{o}$C to obtain (2 alpha, 3 beta,-4 alpha)-($\pm$)-3,4-dihydro-2-methyl-4-(methylamino)-2H-1-benzopyran-3-ol, hydrogen sulphate ester having the formula V:

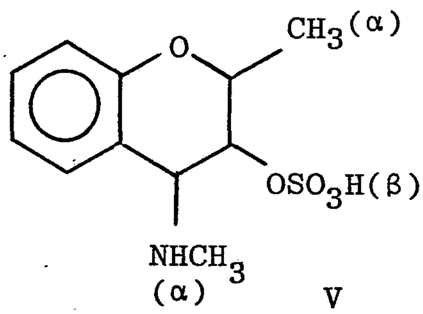

V

5. heating compound V and an alkaline metal hydroxide to obtain (1a alpha, 2 beta,-7b alpha)-($\pm$)-1,1a,3,7b-tetrahydro-1,2-dimethyl[1]benzopyrano[3,4-b]azirine having the formula VI:

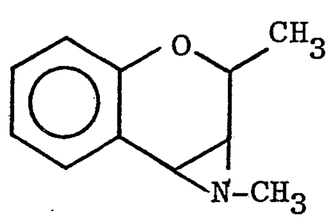

cis

VI

and,

6. subjecting a solution of compound VI to catalytic hydrogenation to effect ring

opening and formation of compound VII.

3. A process for producing cis-(±)-3,4-dihydro-N,2-dimethyl-2H-1-benzopyran-3-amine having the formula VII:

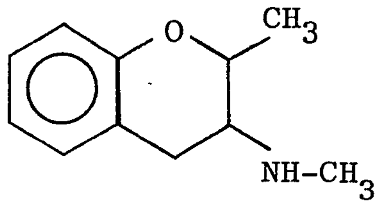

cis

VII

which process comprises the following steps 4 to 6:

4. reacting sulphuric acid in an organic solvent with (2 alpha, 3 beta, 4 alpha)-(±)-3,4-dihydro-2-methyl-4-(methylamino)-2H-1-benzopyran-3-ol having the formula IV:

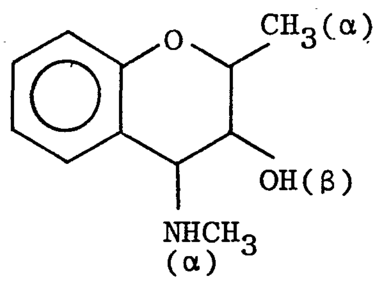

IV

at a temperature above 110$^{o}$C to obtain (2 alpha, 3 beta, 4 alpha)-(±)-3,4-dihydro-2-methyl-4-(methylamino)-2H-1-benzopyran-3-ol, hydrogen sulphate ester having the formula V:

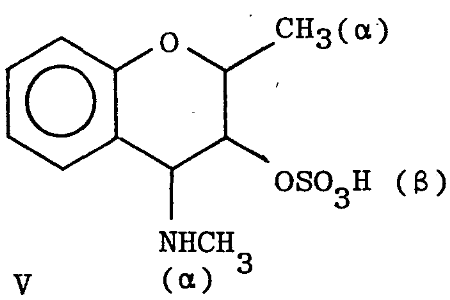

V

5. heating compound V and an alkaline metal hydroxide to obtain (1a alpha, 2 beta, 7b-alpha)-(±)-1,1a,2,7b-tetrahydro-1,2-dimethyl[1]benzopyrano[3,4-b]azirine having the formula VI:

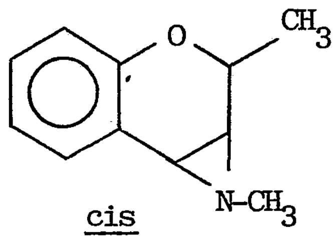

cis

VI

6. subjecting a solution of compound VI to catalytic hydrogenation to effect ring opening and formation of compound VII.

4. A compound having the formula VIII:

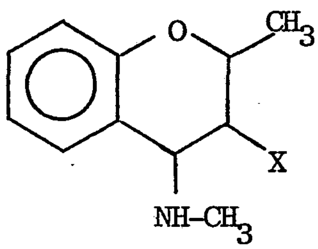

2α, 3β, 4α-(±)    VIII

wherein X represents -OH or $-OSO_3H$.

5. (1a alpha, 3 beta, 7b alpha)-(±)-1,1a,2,7b-tetrahydro-1,2-dimethyl[1]benzopyrano[3,4-b]azirine having the formula VI:

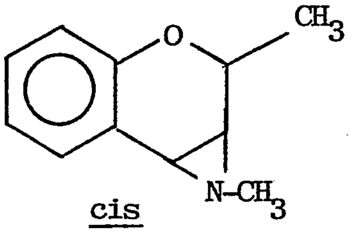

cis

VI

6. A process for producing (1a alpha, 2 beta,-7b alpha)-(±)-1,1a,2,7b-tetrahydro-1,2-dimethyl[1]-benzopyrano[3,4-b]azirine having the formula VI:

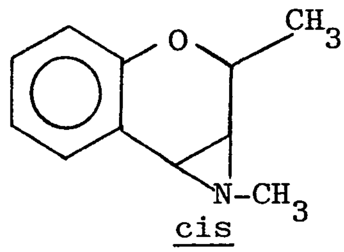

VI

which process comprises the following steps:

1. reacting a solution of 2-methyl-2H-1-benzopyran having the formula II:

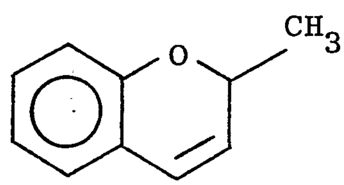

II

in water and/or in a nonreactive, water-miscible solvent, with bromine to obtain, in situ, 3,4-dibromo-3,4-dihydro-2-methyl-2H-1-benzopyran having the formula IIa:

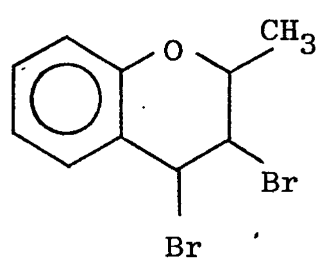

11a

2. treating the reation mixture produced in Step 1 with water and neutralizing with an alkali to obtain 3,4-dihydro-3-bromo-2-methyl-2H-1-benzopyran-4-ol having the formula III:

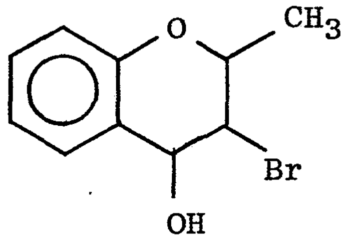

III

3. reacting compound III in water or a water-miscible solvent with an aqueous solution of methylamine to form, in situ, 3,4-epoxy-2-methylbenzopyran having the formula IIIa:

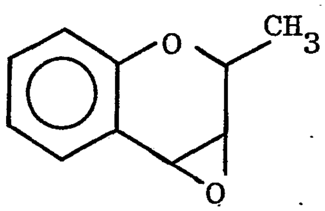

IIIa

and continuing the reaction to form (2 alpha, 3 beta, 4 alpha)-(±)-3,4-dihydro-2-methyl-4-(methylamino)-2H-1-benzopyran-3-ol having the formula IV:

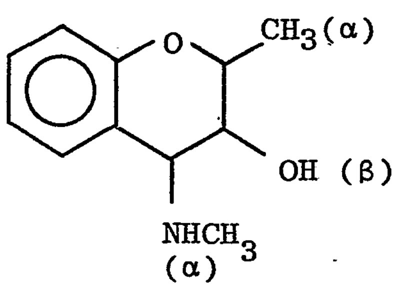

IV

4. reacting sulphuric acid in an organic solvent with compound IV at a temperature above 110°C to obtain (2 alpha, 3 beta,-4 alpha)-(±)-3,4-dihydro-2-methyl-4-(methylamino)-2H-1-benzopyran-3-ol,

hydrogen sulphate ester having the formula V:

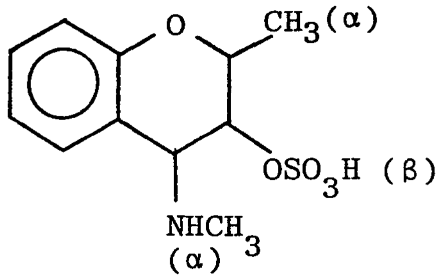

V

and,

5. heating compound V and an alkaline metal hydroxide to obtain (1a alpha, 2 beta, -7b alpha)-(±)-1,1a,2,7b-tetrahydro-1,2-dimethyl[1]benzopyrano[3,4-b]azirine having the formula VI.

7. A process for producing (2 alpha, 3 beta, 4 alpha) -(±)-3,4-dihydro-2-methyl-4-(methylamino)-2H-1-benzopyran -3-ol, hydrogen sulphate ester having the formula V:

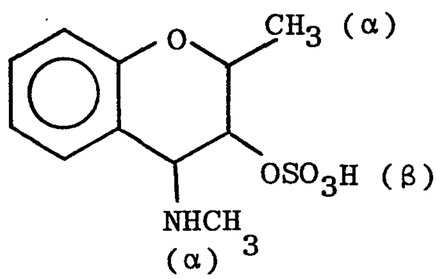

V

which process comprises the following steps:

1. reacting a solution of 2-methyl-2H-1-benzopyran having the formula II:

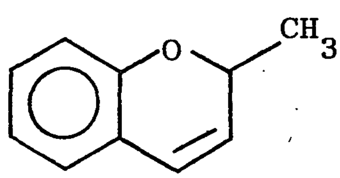

II

in water and/or in a non-reactive, water-miscible solvent, with bromine to obtain, in situ, 3,4-dibromo-3,4-dihydro-2-methyl-2H-1-benzopyran having the formula IIa:

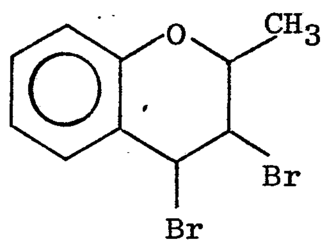

IIa

2. treating the reaction mixture produced in Step 1 with water and neutralizing with an alkali to obtain 3,4-dihydro-3-bromo-2-methyl-2H-1-benzopyran-4-ol having the formula III:

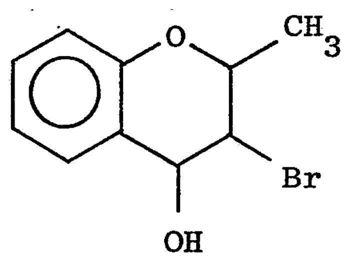

III

3. reacting compound III in water or a water-miscible solvent with an aqueous solution of methylamine to form, in situ, 3,4-epoxy-2-methylbenzopyran having the formula IIIa:

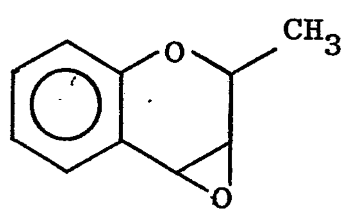

IIIa

and continuing the reaction to form (2 alpha, 3 beta, 4 alpha)-(±)-3,4-dihydro-2-methyl-4-(methylamino)-2H-1-benzopyran-3-ol having the formula IV:

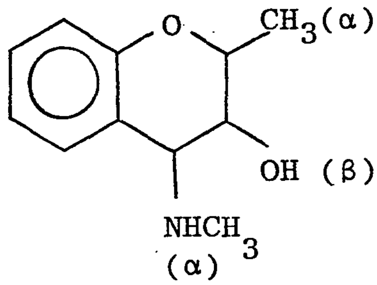

IV

and,

4. reacting sulphuric acid in an organic solvent with compound IV at a temperature above 110°C to obtain (2 alpha, 3 beta, 4 alpha)-(±)-3,4-dihydro-2-methyl-4-(methylamino)-2H-1-benzopyran-3-ol, hydrogen sulphate ester having the formula V.

8. A process for producing (2 alpha, 3 beta, 4 alpha)-(±)-3,4-dihydro-2-methyl-4-(methylamino)-2H-1-benzopyran-3-ol having the formula IV:

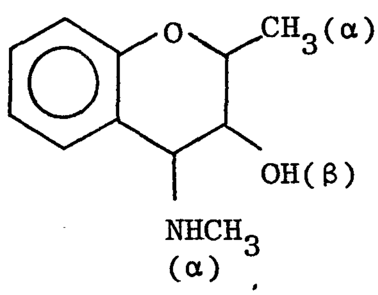

IV

which process comprises the following steps:

1. reacting a solution of 2-methyl-2H-1-benzopyran having the formula II:

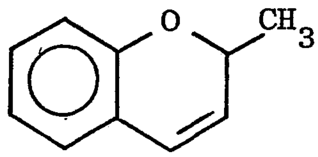

II

in water and/or in a nonreactive, water-miscible solvent with bromine to obtain, in situ, 3,4-dibromo-3,4-dihydro-2-methyl-2H-1-benzopyran having the formula IIa:

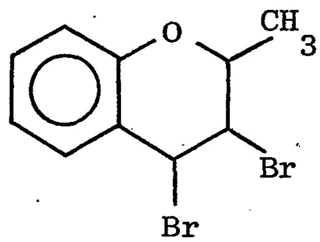

IIa

2. treating the reaction mixture produced in Step 1 with water and neutralizing with an alkali to obtain 3,4-dihydro-3-bromo-2-methyl-2H-1-benzopyran-4-ol having the formula III:

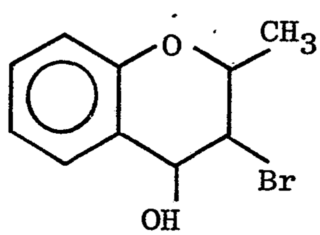

III

and,

3. reacting compound III in water or a water-miscible solvent with an aqueous solution of

methylamine to form, in situ, 3,4-epoxy-2-methylbenzopyran having the formula IIIa:

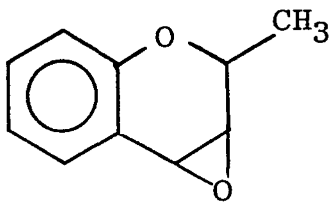

IIIa

and continuing the reaction to form (2 alpha, 3 beta, 4 alpha)-(±)-3,4-dihydro-2-methyl-4-(methylamino)-2H-1-benzopyran-3-ol having the formula IV.

9. A process for producing cis-(±)-3,4-dihydro-N,N,-2-trimethyl-2H-1-benzopyran-3-amine having the formula I:

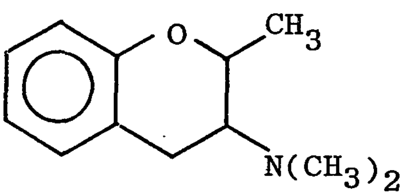

cis

I

or a non-toxic pharmaceutically-acceptable acid-addition salt thereof, which process comprises methylating a compound of the formula VII

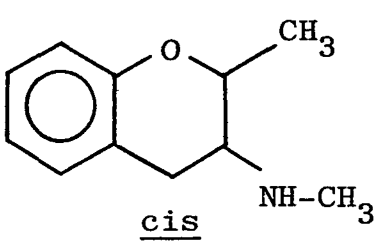

cis

VII

to obtain compound I in the form of a free base or a

non-toxic pharmaceutically acceptable acid-addition salt thereof.

10. cis-(±)-3,4-dihydro-N,2-dimethyl-2H-1-benzopyran-3-amine having the formula VII:-

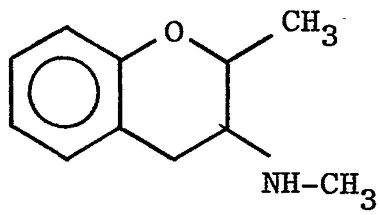

cis

VII .

European Patent Office

# EUROPEAN SEARCH REPORT

0027835

Application number

EP 79302262.5

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| D,X | US - A - 3 607 886 (LOCKHART)<br>+ Totality +<br>-- | 1,9,10 | C 07 D 311/58<br>C 07 D 311/68<br>C 07 D 491/052//<br>(C 07 D 491/052<br>C 07 D 203/00<br>C 07 D 311/00) |
| | US - A - 4 110 347 (WATTS)<br>+ Columns 4,5; claims +<br>-- | 1-4, 6-8 | |
| | US - A - 4 115 407 (WRIGHT)<br>+ Claims +<br>-- | 1-4, 6-8 | |
| | US - A - 4 119 643 (WATTS)<br>+ Columns 4,5; claims +<br>---- | 1-4, 6-8 | |
| | | | TECHNICAL FIELDS SEARCHED (Int.Cl. 3) |
| | | | C 07 D 311/00<br>C 07 D 491/00 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 10-04-1980 | HAMMER |

EPO Form 1503.1 06.78